# EUROPEAN PATENT APPLICATION

(11) **EP 2 168 982 A1**
(43) Date of publication of application: **31.03.2010**
(21) Application number: 09160182.3
(22) Date of filing: 15.12.2005
(51) Int. Cl.: C07K 14/50, C07K 14/605, A61K 38/26, A61P 3/10, C12N 15/62, C07K 14/435, A61K 38/00

(54) **GLP-1 analog fusion protein formulations**

(30) Priority: 22.12.2004 US 641690 P
(62) Divisional of application: 05854150.9
(71) Applicant: Eli Lilly & Company, Indianapolis, IN 46285 (US)
(72) Inventor: Glaesner, Wolfgang, San Diego, CA 92130 (US); Millican, Rohn Lee Junior, Indianapolis, IN 46259 (US)
(74) Representative: Kent, Lindsey Ruth

(57) **Abstract**

The invention provides a stable solution formulation comprising a GLP-1-Fc fusion at a pH between about pH 6 and about pH 8.5. analogs fused to specific IgG4-Fc derivatives. These formulations provide unexpected and considerably greater chemical stability than when compared to GLP-1-Fc fusions at a pH outside the described ranges. The formulations comprising a GLP-1-Fc fusion are useful in treating diabetes, obesity, irritable bowel syndrome and other conditions that would be benefited by lowering plasma glucose, inhibiting gastric and/or intestinal motility and inhibiting gastric and/or intestinal emptying, or inhibiting food intake.

## Description

### FIELD OF THE INVENTION

The present invention relates to formulations of glucagon-like peptide analogs fused to proteins that have the effect of stabilizing the fusion proteins. These formulations can be used to treat diabetes as well as a variety of other conditions or disorders.

### BACKGROUND OF THE INVENTION

Glucagon-like peptide-1 (GLP-1) analogs and derivatives show promise in clinical trials for the treatment of type 2 diabetes. GLP-1 induces numerous biological effects such as stimulating insulin secretion, inhibiting glucagon secretion, inhibiting gastric emptying, inhibiting gastric motility or intestinal motility, and inducing weight loss. A significant characteristic of GLP-1 is its ability to stimulate insulin secretion without the associated risk of hypoglycemia that is seen when using insulin therapy or some types of oral therapies that act by increasing insulin expression.

The usefulness of therapy involving GLP-1 peptides has been limited by the fact that GLP-1(1-37) is poorly active, and the two naturally occurring truncated peptides, GLP-1(7-37)OH and GLP-1(7-36)NH2, are rapidly cleared *in vivo* and have extremely short *in vivo* half lives. It is known that endogenously produced dipeptidyl-peptidase IV (DPP-IV) inactivates circulating GLP-1 peptides by removing the N-terminal histidine and alanine residues and is a major reason for the short *in vivo* half-life.

Various approaches have been undertaken to extend the elimination half-life of a GLP-1 peptide or reduce clearance of the peptide from the body while maintaining biological activity. One approach involves fusing a GLP-1 peptide to the Fc portion of an immunoglobulin. Immunoglobulins typically have long circulating half-lives *in vivo.*

For example, IgG molecules can have a half-life in humans of up to 23 days. The Fc portion of the immunoglobulin is responsible, in part, for this *in vivo* stability. GLP-1-Fc fusion proteins take advantage of the stability provided by the Fc portion of an immunoglobulin while preserving the biological activity of the GLP-1 molecule.

Although this approach is feasible for GLP-1 therapeutics (See WO 02/46227), there is a general concern regarding the antigenicity of various fusion proteins when administered repeatedly over prolonged periods of time. This is especially a concern for GLP-1-Fc fusion therapeutics as a patient with diabetes must be treated for her entire life once diagnosed with the disease. In addition, Fc fusion protein therapeutics can be a concern if the Fc portion retains unwanted effector functions. This approach is the focus of PCT/US 04/15595 (WO2005/000892), in which problems associated with the potential immunogenicity and effector activity associated with administration of GLP-1-Fc fusions are overcome by identifying specific GLP-1-Fc fusion proteins that have a reduced risk of inducing an immune response after repeated and prolonged administration and no longer have effector function.

The fusion proteins of this nature are technically too large and complex to produce synthetically or recombinantly in bacterial cells. These fusion proteins are typically produced in mammalian cells, such as CHO, 293, or NSO. It was observed that the fusion proteins produced in mammalian cells where more readily susceptible to degradation by endogenous proteases and chemical alteration than non-fusion proteins produced in bacterial cells. This problem was sought to be overcome in the present invention. It is discovered that a formulation comprising a GLP-1-Fc fusion buffered between about pH 6 and about pH 8.5 provided increased chemical stability.

### SUMMARY OF THE INVENTION

In order to overcome the problem of chemical stability of GLP-1-Fc fusions, the present inventors have developed a stable solution formulation. In particular, the inventors have discovered that a formulation comprising a therapeutically effective amount of a GLP-1-Fc fusion at a pH between about pH 6 and about pH 8.5, preferably between about pH 6 and about pH 7.5, between about pH 6 and about pH 7, between about pH 6.5 and about pH 7.5, or between about pH 6 and about pH 6.5, and even more preferably, about pH 6 or about 6.5, provided unexpectedly and considerably greater chemical stability than when compared to GLP-1-Fc fusions at a pH outside the described ranges.

The present invention also includes methods of treating patients suffering from non-insulin dependent as well as insulin dependent diabetes mellitus, obesity, and various other disorders and conditions comprising administering the formulations of GLP-1-Fc fusions.

### DETAILED DESCRIPTION OF THE INVENTION

The fusion proteins comprise a GLP-1 compound fused to an Fc portion of an immunoglobulin, an analog of an Fc portion of an immunoglobulin, or a fragment of an Fc portion of an immunoglobulin. The C-terminus of the GLP-1 compound may be fused directly, or fused via a peptide linker, to the N-terminus of an Fc protein. These fusion proteins are biologically active and have an increased half-life compared to native GLP-1. The GLP-1 compounds that make up part of the fusion protein encompass polypeptides having from about twenty-five to about thirty-nine naturally occurring or non-naturally occurring amino acids that have sufficient homology to native GLP-1(7-37)OH such that they exhibit insulinotropic activity by binding to the GLP-1 receptor on β-cells in the pancreas. A GLP-1 compound typically comprises a polypeptide having the amino acid sequence of GLP-1(7-37)OH, an analog of GLP-1 (7-37)OH, a fragment of GLP-1(7-37)OH or a fragment of a GLP-1(7-37)OH analog.

Examples of fusion proteins useful in the formulations include fusion proteins comprising a GLP-1 analog comprising a sequence selected from the group consisting of
a) 
(SEQ ID NO:1)
His-Xaa8-Glu-Gly-Thr-Phe-Thr-Ser-Asp-Val-Ser-Ser-Tyr-Leu-Glu-Glu-
Gln-Ala-Ala-Lys-Glu-Phe-Ile-Ala-Trp-Leu-Val-Lys-Gly-Gly-Gly
 wherein Xaa₈ is selected from Gly and Val;
b) 
(SEQ ID NO:2)
His-Xaa8-Glu-Gly-Thr-Phe-Thr-Ser-Asp-Val-Ser-Ser-Tyr-Leu-Glu-Glu-
Gln-Ala-Ala-Lys-Glu-Phe-Ile-Ala-Trp-Leu-Lys-Asn-Gly-Gly-Gly
 wherein Xaa₈ is selected from Gly and Val;
c) 
(SEQ ID NO:3)
His-Xaa8-Glu-Gly-Thr-Phe-Thr-Ser-Asp-Val-Ser-Ser-Tyr-Leu-Glu-Glu-
Gln-Ala-Ala-Lys-Glu-Phe-Ile-Ala-Trp-Leu-Val-Lys-Gly-Gly-Pro
 wherein Xaa₈ is selected from Gly and Val;
d) 
(SEQ ID NO:4)
His-Xaa8-Glu-Gly-Thr-Phe-Thr-Ser-Asp-Val-Ser-Ser-Tyr-Leu-Glu-Glu-
Gln- Ala-Ala-Lys-Glu-Phe-Ile-Ala-Trp-Leu-Lys-Asn-Gly-Gly-Pro
 wherein Xaa₈ is selected from Gly and Val;
e) (SEQ ID NO:5) 
His-Xaa8-Glu-Gly-Thr-Phe-Thr-Ser-Asp-Val-Ser-Ser-Tyr-Leu-Glu-Glu-
Gln-Ala-Ala-Lys-Glu-Phe-Ile-Ala-Trp-Leu-Val-Lys-Gly-Gly
 wherein Xaa₈ is selected from Gly and Val;
f) 
(SEQ ID NO:6)
His-Xaa8-Glu-Gly-Thr-Phe-Thr-Ser-Asp-Val-Ser-Ser-Tyr-Leu-Glu-Glu-
Gln-Ala-Ala-Lys-Glu-Phe-Ile-Ala-Trp-Leu-Lys-Asn-Gly-Gly
 wherein Xaa₈ is selected from Gly and Val;
fused to the Fc portion of an immunoglobulin comprising the sequence of SEQ ID NO:7 
Ala-Glu-Ser-Lys-Tyr-Gly-Pro-Pro-Cys-Pro-Pro-Cys-Pro-Ala-Pro-
Xaa16-Xaa17-Xaa18-Gly-Gly-Pro-Ser-Val-Phe-Leu-Phe-Pro-Pro-Lys-Pro-
Lys-Asp-Thr-Leu-Met-Ile-Ser-Arg-Thr-Pro-Glu-Val-Thr-Cys-Val-
Val-Val-Asp-Val-Ser-Gln-Glu-Asp-Pro-Glu-Val-Gln-Phe-Asn-Trp-
Tyr-Val-Asp-Gly-Val-Glu-Val-His-Asn-Ala-Lys-Thr-Lys-Pro-Arg-
Glu-Glu-Gln-Phe-Xaa80-Ser-Thr-Tyr-Arg-Val-Val-Ser-Val-Leu-Thr-
Val-Leu-His-Gln-Asp-Trp-Leu-Asn-Gly-Lys-Glu-Tyr-Lys-Cys-Lys-
Val-Ser-Asn-Lys-Gly-Leu-Pro-Ser-Ser-Ile-Glu-Lys-Thr-Ile-Ser-
Lys-Ala-Lys-Gly-Gln-Pro-Arg-Glu-Pro-Gln-Val-Tyr-Thr-Leu-Pro-
Pro-Ser-Gln-Glu-Glu-Met-Thr-Lys-Asn-Gln-Val-Ser-Leu-Thr-Cys-
Leu-Val-Lys-Gly-Phe-Tyr-Pro-Ser-Asp-Ile-Ala-Val-Glu-Trp-Glu-
Ser-Asn-Gly-Gln-Pro-Glu-Asn-Asn-Tyr-Lys-Thr-Thr-Pro-Pro-Val-
Leu-Asp-Ser-Asp-Gly-Ser-Phe-Phe-Leu-Tyr-Ser-Arg-Leu-Thr-Val-
Asp-Lys-Ser-Arg-Trp-Gln-Glu-Gly-Asn-Val-Phe-Ser-Cys-Ser-Val-
Met-His-Glu-Ala-Leu-His-Asn-His-Tyr-Thr-Gln-Lys-Ser-Leu-Ser-
Leu-Ser-Leu-Gly-Xaa230 (SEQ ID NO:7)
 wherein:
Xaa at position 16 is Pro or Glu;
Xaa at position 17 is Phe, Val, or Ala;
Xaa at position 18 is Leu, Glu, or Ala;
Xaa at position 80 is Asn or Ala; and
Xaa at position 230 is Lys or is absent.

The C-terminus of the GLP-1 analog portion and the N-terminus of the Fc portion of the fusion proteins are preferably fused together via 1, 1.5 or 2 repeats of a G-rich peptide linker having the sequence Gly-Gly-Gly-Gly-Ser-Gly-Gly-Gly-Gly-Ser-Gly-Gly-Gly-Gly-Ser (SEQ ID NO:8).

The fusion proteins useful in the formulations of the present invention comprise a GLP-1 analog portion and an Fc portion. The GLP-1 analog portion and the Fc portion comprise substitutions to the native GLP-1 sequence and the human IgG4 sequence respectively that provide the protein with increased potency and *in vivo* stability compared to native GLP-1 or GLP-1 analogs not fused to an Fc sequence while decreasing the potential for inducing antibody formation after prolonged and repeated administration in humans.

Native GLP-1 is processed *in vivo* such that the first 6 amino acids are cleaved from the molecule. Thus, by custom in the art, the amino terminus of GLP-1 has been assigned the number 7 and the carboxy-terminus, number 37. The other amino acids in the polypeptide are numbered consecutively as shown in SEQ ID NO:9. For example, position 8 is alanine and position 22 is glycine. The processed peptide may be further modified *in vivo* such that the C-terminal glycine residue is removed and replaced with an amide group. Thus, GLP-1(7-37)OH and GLP-1(7-36)amide represent the two native forms of the molecule. GLP-1(7-37)OH has the amino acid sequence of SEQ ID NO:9:

⁷His-Ala-Glu-¹⁰Gly-Thr-Phe-Thr-Ser-¹⁵Asp-Val-Ser-Ser-Tyr-²⁰Leu-Glu-Gly-
Gln-Ala-²⁵Ala-Lys-Glu-Phe-Ile-³⁰Ala-Trp-Leu-Val-Lys-³⁵Gly-Arg-³⁷Gly
(SEQ ID NO:9)

The GLP-1 analog portion of the fusion protein comprises three primary substitutions at positions 8, 22, and 36 relative to native GLP-1(7-37). The substitution at position 8 reduces the rate at which the endogenous enzyme dipeptidyl-peptidase IV (DPP-IV) inactivates the analog. DPP-IV cleaves native GLP-1 between the 2^{nd} and 3^{rd} amino acids (between position 8 and 9) and the resulting molecule is less active. Thus, the fusion proteins useful in the formulations of the present invention are DPP-IV resistant. The substitution at position 22 reduces the potential of the molecule to aggregate and increases the potency of the molecule. The substitution at position 36 in the context of the analog with changes at 8 and 22 as well as in the context of the entire fusion protein reduces the risk that the fusion protein will induce a neutralizing immune response after repeated and prolonged administration in humans.

The GLP-1 analog C-terminus is preferably one of the following sequences: Trp-Leu-Val-Lys-Gly-Gly-Gly (SEQ ID NO: 10); Trp-Leu-Lys-Asn-Gly-Gly-Gly (SEQ ID NO:11); Trp-Leu-Val-Lys-Gly-Gly-Pro (SEQ ID NO:12); Trp-Leu-Lys-Asn-Gly-Gly-Pro (SEQ ID NO: 13); Trp-Leu-Val-Lys-Gly-Gly (SEQ ID NO:14); and Trp-Leu-Lys-Asn-Gly-Gly (SEQ ID NO: 15).

The fusion proteins useful in the formulations of the present invention contain an Fc portion which is derived from human IgG4, but comprises one or more substitutions compared to the wild-type human sequence. As used herein, the Fc portion of an immunoglobulin has the meaning commonly given to the term in the field of immunology. Specifically, this term refers to an antibody fragment which does not contain the two antigen binding regions (the Fab fragments) from the antibody. The Fc portion consists of the constant region of an antibody from both heavy chains, which associate through non-covalent interactions and disulfide bonds. The Fc portion can include the hinge regions and extend through the CH2 and CH3 domains to the c-terminus of the antibody. The Fc portion can further include one or more glycosylation sites.

There are five types of human immunoglobulins with different effector functions and pharmcokinetic properties. IgG is the most stable of the five types having a serum half-life in humans of about 23 days. There are four IgG subclasses (G1, G2, G3, and G4) each of which have different biological functions known as effector functions. These effector functions are generally mediated through interaction with the Fc receptor (FcγR) or by binding C1q and fixing complement. Binding to FcγR can lead to antibody dependent cell mediated cytolysis, whereas binding to complement factors can lead to complement mediated cell lysis. In designing Fc fusion proteins wherein the Fc portion is being utilized solely for its ability to extend half-life, it is important to minimize any effector function. Thus, the fusion proteins useful in the formulations of the present invention are derived from the human IgG4 Fc region because of its reduced ability to bind FcγR and complement factors compared to other IgG sub-types. IgG4, however, has been shown to deplete target cells in humans [Issacs et al., (1996) Clin. Exp. Immunol. 106:427-433]. Because the fusion proteins target beta cells in the pancreas to induce insulin expression, using an IgG4 derived region in an Fc fusion protein could initiate an immune response against the pancreateic beta cell through interaction of the fusion protein with the GLP-1 receptor present on pancreatic beta cells. Thus, the IgG4 Fc region which is part of the fusion proteins contains substitutions that eliminate effector function. The IgG4 Fc portion of the fusion proteins may contain one or more of the following substitutions: substitution of proline for glutamate at residue 233, alanine or valine for phenylalanine at residue 234 and alanine or glutamate for leucine at residue 235 (EU numbering, Kabat, E.A. et al. (1991) Sequences of Proteins of Immunological Interest, 5th Ed. U.S. Dept. of Health and Human Services, Bethesda, MD, NIH Publication no. 91-3242). These residues corresponds to positions 16, 17 and 18 in SEQ ID NO:7. Further, removing the N-linked glycosylation site in the IgG4 Fc region by substituting Ala for Asn at residue 297 (EU numbering) which corresponds to position 80 of SEQ ID NO:7 is another way to ensure that residual effector activity is eliminated in the context of a fusion protein.

In addition, the IgG4 Fc portion of the fusion proteins contain a substitution that stabilizes heavy chain dimer formation and prevents the formation of half-IgG4 Fc chains. The fusion proteins preferably exist as dimers joined together by disulfide bonds and various non-covalent interactions. Wild-type IgG4 contains a Pro-Pro-Cys-Pro-Ser-Cys (SEQ ID NO: 16) motif beginning at residue 224 (EU numbering). This motif in a single GLP-1 analog-Fc chain forms disulfide bonds with the corresponding motif in another GLP-1 analog-Fc chain. However, the presence of serine in the motif causes the formation of single chain fusion proteins. The present invention encompasses Fc fusion proteins wherein the IgG4 sequence is further modified such that serine at position at 228 (EU numbering) is substituted with proline (amino acid residue 11 in SEQ ID NO:7).

The C-terminal lysine residue present in the native molecule may be deleted in the IgG4 derivative Fc portion of the fusion proteins discussed herein (position 230 of SEQ ID NO:7; deleted lysine referred to as des-K). Fusion proteins expressed in some cell types (such as NS0 cells) wherein lysine is encoded by the C-terminal codon are heterogeneous in that a portion of the molecules have lysine as the C-terminal amino acid and a portion have lysine deleted. The deletion is due to protease action during expression in some types of mammalian cells. Thus, to avoid this heterogeneity, it is preferred that Fc fusion expression constructs lack a C-terminal codon for lysine.

It is preferred that the C-terminal amino acid of the GLP-1 analog portion discussed herein is fused to the N-terminus of the IgG4 Fc analog portion via a glycine-rich linker. The *in vivo* function and stability of the fusion proteins can be optimized by adding small peptide linkers to prevent potentially unwanted domain interactions. Further, a glycine-rich linker provides some structural flexibility such that the GLP-1 analog portion can interact productively with the GLP-1 receptor on target cells such as the beta cells of the pancreas. These linkers, however, can significantly increase the risk that the fusion protein will be immunogenic *in vivo.* Thus, it is preferred that the length be no longer than necessary to prevent unwanted domain interactions and/or optimize biological activity and/or stability. The preferred glycine-rich linker comprises the sequence: Gly-Gly-Gly-Gly-Ser-Gly-Gly-Gly-Gly-Ser-Gly-Gly-Gly-Gly-Ser (SEQ ID NO:8). Although more copies of this linker may be used in the fusion proteins, it is preferred that a single copy of this linker be used to minimize the risk of immunogenicity associated with prolonged and repeated administration.

Preferred GLP-1-Fc fusions include the following proteins: Gly⁸-Glu²2-Gly³⁶-GLP-1(7-37)-1L-IgG4 (S228P), Gly⁸-Glu²²-Gly³⁶-GLP-1(7-37)-1L-IgG4 (S228P, F234A, L235A), Gly⁸-Glu²²-Gly³⁶-GLP-1(7-37)-1L-IgG4 (S228P, N297A), Gly⁸-Glu²²-Gly³⁶-GLP-1(7-37)-1L-IgG4 (S228P, F234A, L235A, N297A), Gly⁸-Glu²²-Gly³⁶-GLP-1(7-37)-1.5L-IgG4 (S228P), Gly⁸-Glu²²-Gly³⁶-GLP-1(7-37)-1.5L-IgG4 (S228P, F234A, L235A), Gly⁸-Glu²²-Gly³⁶-GLP-1(7-37)-1.5L-IgG4 (S228P, N297A), Gly⁸-Glu²²-Gly³⁶-GLP-1(7-37)-1.5L-IgG4 (S228P, F234A, L235A, N297A), Gly⁸-Glu²²-Gly³⁶-GLP-1(7-37)-2L-IgG4 (S228P), Gly⁸-Glu²²-Gly³⁶-GLP-1(7-37)-2L-IgG4 (S228P, F234A, L235A), Gly⁸-Glu²²-Gly³⁶-GLP-1(7-37)-2L-IgG4 (S228P, N297A), and Gly⁸-Glu²²-Gly³⁶-GLP-1(7-37)-2L-IgG4 (S228P, F234A, L235A, N297A), and the Val⁸ and des-K forms of all of the above.

The nomenclature used herein to refer to specific fusion proteins is defined as follows: Specific substitutions to the GLP-1 portion of the fusion protein are indicated using the specific amino acid being substituted followed by the residue number. GLP-1(7-37) indicates that the GLP-1 portion of the mature fusion protein begins with His at position 7 and ends with Gly at position 37. L refers to a linker with the sequence Gly-Gly-Gly-Gly-Ser-Gly-Gly-Gly-Gly-Ser-Gly-Gly-Gly-Gly-Ser (SEQ ID NO:8). The number immediately preceding the L refers to the number of linkers separating the GLP-1 portion from the Fc portion. A linker specified as 1.5L refers to the sequence Gly-Ser-Gly-Gly-Gly-Gly-Ser-Gly-Gly-Gly-Gly-Ser-Gly-Gly-Gly-Gly-Ser-Gly-Gly-Gly-Gly-Ser (SEQ ID NO: 17). IgG4 refers to an analog of the human IgG4 Fc sequence specified as SEQ ID NO:7. Substitutions in the IgG4 Fc portion of the fusion protein are indicated in parenthesis. The wild-type amino acid is specified by its common abbreviation followed by the position number in the context of the entire IgG4 sequence using the EU numbering system followed by the amino acid being substituted at that position specified by its common abbreviation.

The fusion proteins have biological activity. Biological activity refers to the ability of the fusion protein to bind to and activate the GLP-1 receptor *in vivo* and elicit a response. Responses include, but are not limited to, secretion of insulin, suppression of glucagon, inhibition of appetite, weight loss, induction of satiety, inhibition of apoptosis, induction of pancreatic beta cell proliferation, and differentiation of pancreatic beta cells. A representative number of GLP-1 fusion proteins were tested for *in vitro* as well as *in vivo* activity. *In vitro* activity based on the ability of the fusion protein to interact with and activate the human GLP-1 receptor have been shown. (See PCT/US 04/15595 (WO2005/000892)). HEK293 cells over-expressing the human GLP-1 receptor are used. Activation of the GLP-1 receptor in these cells causes adenylyl cyclase activation which in turn induces expression of a reporter gene driven by a cyclic AMP response element (CRE).

The pH of the GLP-1-Fc fusion formulations is adjusted to provide acceptable stability, to maintain the solubility and insulinotropic activity of the GLP-1-Fc fusion and be acceptable for parenteral administration. The pH of the GLP-1-Fc fusion formulations is preferably adjusted to between about pH 6 and about pH 8.5, preferably between about pH 6 and about pH 7.5, between about pH 6 and about pH 7, between about pH 6.5 and about pH 7.5, or between about pH 6 and about pH 6.5, and even more preferably, about pH 6 or about 6.5.

The formulations comprising a GLP-1-Fc fusion of the present invention may optionally encompass a pharmaceutically acceptable buffer. However, the selection and concentration of the buffer shall be such that the formulation can be adjusted to the described ranges that provide acceptable stability and insulinotropic activity. Examples of pharmaceutically acceptable buffers include phosphate buffers like dibasic sodium phosphate, TRIS, acetate, such as sodium acetate, citrate, such as sodium citrate, sodium tartarate, basic amino acids such as histidine, lysine or arginine, or neutral amino acids such as glycine and glycyl-glycine. Other pharmaceutically acceptable buffers are known in the art. Preferably, the buffer is selected from the group consisting of citrate, phosphate and TRIS. The skilled artisan will recognize that the selection of the buffer is dependent upon the described pH ranges and the pKa of the buffer. Preferably, the concentration of a buffer is between about 1 mM and 30 mM. Even more preferably, the concentration is between about 4 mM and 14 mM or between about 5 mM and 20 mM. Even more preferably, the concentration is between about 10 mM and 20 mM. Even more preferably, the concentration is about 10 mM or about 20 mM.

The formulations of the present invention may optionally encompass a preservative. However, the selection and concentration of the preservative shall be such that the formulation can be adjusted to the described ranges that provide acceptable stability and insulinotropic activity. Preservative refers to a compound that is added to a pharmaceutical formulation to act as an anti-microbial agent. A parenteral formulation must meet guidelines for preservative effectiveness to be a commercially viable multi-use product. Among preservatives known in the art as being effective and acceptable in parenteral formulations are phenolic preservatives, alkylparabens, benzyl alcohol, chlorobutanol, resorcinol, and other similar preservatives, and various mixtures thereof. Examples of phenolic derivatives include cresols and phenol or a mixture of cresols and phenol. Examples of cresols include meta-cresol, ortho-cresol, para-cresol, chloro-cresol, or mixtures thereof. Alkylparaben refers to a C₁ to C₄ alkyl paraben, or mixtures thereof. Examples of alkylparabens include methylparaben, ethylparaben, propylparaben, or butylparaben. The concentration of the preservative is known to one skilled in the art. The concentrations must be sufficient to maintain preservative effectiveness by retarding microbial growth.

The preferred preservative is meta-cresol or phenol. In general, the concentration of meta-cresol is between about 2.0 to about 8.0 mg/mL, about 2.5 mg/mL to about 4.5 mg/mL, and about 2.0 mg/mL to about 4.0 mg/mL. A most preferred concentration of preservative in the formulation is about 2.7 mg/mL. In another embodiment, the concentration of phenol is between about 2.0 to about 10.0 mg/mL, and about 4.0 to about 8.0 mg/mL. A most preferred concentration of preservative in the formulation is about 5.0 mg/mL.

The formulations of the present invention may optionally encompass an isotonicity agent. However, the selection and concentration of the isotonicity agent shall be such that the formulation can be adjusted to the described ranges that provide acceptable stability and insulinotropic activity. Isotonicity agents refer to compounds that are tolerated physiologically and impart a suitable tonicity to the formulation to prevent the net flow of water across cell membranes. Examples of such compounds include glycerin (or glycerol), salts, e.g., NaCl, and sugars, e.g., dextrose, mannitol, and sucrose. These compounds are commonly used for such purposes at known concentrations. One or more isotonicity agents may be added to adjust the ionic strength or tonicity.

The preferred isotonicity agent is NaCl. The concentration of NaCl is preferably between about 10 mM and 500 mM, more preferred is between about 50 mM and 200 mM, and most preferred is about 150 mM. In another embodiment, the preferred isotonicity agent is mannitol. The concentration of the mannitol is preferably between about 1% (weight (w)/volume (v)) and 10% (w/v), and more preferred is between about 2% (w/v) and 8% (w/v). In another embodiment, the preferred isotonicity agent is glycerin. The concentration of the glycerin is preferably between about 12 mg/mL and 25 mg/ml, preferably between about 12 mg/mL and 20 mg/ml, and more preferred is about 17 mg/ml.

The formulations of the present invention may optionally encompass a solubility enhancer. However, the selection and concentration of the solubility enhancer shall be such that the formulation can be adjusted to the described ranges that provide acceptable stability and insulinotropic activity. Solubility enhancers provide stability such that the GLP-1-Fc fusion remains soluble for an extended period of time under the conditions of storage. Preferably the solubility enhancer is nicotinamide. In general, the concentration of nicotinamide is between 0.01 and 2 molar. Other preferred ranges of nicotinamide concentration are: between 0.05 molar and 1.5 molar; between 0.1 molar and 1.0 molar; between 0.1 molar and 0.5 molar; between 0.5 molar and 1.0 molar; and between 0.15 molar and 0.25 molar.

Other additives, such as a pharmaceutically acceptable solubilizers like Tween 20^{®} (polyoxyethylene (20) sorbitan monolaurate), Tween 40^{®} (polyoxyethylene (20) sorbitan monopalmitate), Tween 80^{®} (polyoxyethylene (20) sorbitan monooleate), Pluronic F68^{®} (polyoxyethylene polyoxypropylene block copolymers), and PEG (polyethylene glycol) may optionally be added to the formulation. Preferably the solubilizer is Tween 20^{®} or Tween80^{®}. In general, the concentration of Tween 20^{®} or Tween80^{®} is between 0.001% and 0.05%. Other preferred ranges of Tween 20^{®} or Tween80^{®} concentrations are: between 0.005% and 0.05%; between 0.0075% and 0.05%; and between 0.01% and 0.05%.

Administration of the formulations may be via any route known to be effective by the physician of ordinary skill. Peripheral parenteral is one such method. Parenteral administration is commonly understood in the medical literature as the injection of a dosage form into the body by a sterile syringe or some other mechanical device such as an infusion pump. Peripheral parenteral routes can include intravenous, intramuscular, subcutaneous, and intraperitoneal routes of administration.

The formulations may also be amenable to administration by oral, rectal, nasal, or lower respiratory routes, which are non-parenteral routes. Of these non-parenteral routes, the lower respiratory route and the oral route are preferred.

The formulations comprising a GLP-1-Fc fusion can be used to treat a wide variety of diseases and conditions. The GLP-1-Fc fusions primarily exert their biological effects by acting at a receptor referred to as the "GLP-1 receptor." Subjects with diseases and/or conditions that respond favorably to GLP-1 receptor stimulation or to the administration of GLP-1 compounds can therefore be treated with the GLP-1-Fc fusions. These subjects are said to "be in need of treatment with GLP-1 compounds" or "in need of GLP-1 receptor stimulation". Included are subjects with non-insulin dependent diabetes, insulin dependent diabetes, stroke (see WO 00/16797), myocardial infarction (see WO 98/08531), obesity (see WO 98/19698), catabolic changes after surgery (see U.S. Patent No. 6,006,753), functional dyspepsia and irritable bowel syndrome (see WO 99/64060). Also included are subjects requiring prophylactic treatment with a GLP-1 compound, e.g., subjects at risk for developing non-insulin dependent diabetes (see WO 00/07617). Subjects with impaired glucose tolerance or impaired fasting glucose, subjects whose body weight is about 25% above normal body weight for the subject's height and body build, subjects with a partial pancreatectomy, subjects having one or more parents with non-insulin dependent diabetes, subjects who have had gestational diabetes and subjects who have had acute or chronic pancreatitis are at risk for developing non-insulin dependent diabetes.

An effective amount of the GLP-1-Fc fusions in the context of the described formulations is the quantity which results in a desired therapeutic and/or prophylactic effect without causing unacceptable side-effects when administered to a subject in need of GLP-1 receptor stimulation. A "desired therapeutic effect" includes one or more of the following: 1) an amelioration of the symptom(s) associated with the disease or condition; 2) a delay in the onset of symptoms associated with the disease or condition; 3) increased longevity compared with the absence of the treatment; and 4) greater quality of life compared with the absence of the treatment. For example, an "effective amount" of a GLP-1-Fc fusion for the treatment of diabetes is the quantity that would result in greater control of blood glucose concentration than in the absence of treatment, thereby resulting in a delay in the onset of diabetic complications such as retinopathy, neuropathy or kidney disease. An "effective amount" of a GLP-1-Fc fusion for the prevention of diabetes is the quantity that would delay, compared with the absence of treatment, the onset of elevated blood glucose levels that require treatment with anti-hypoglycaemic drugs such as sulfonyl ureas, thiazolidinediones, insulin and/or bisguanidines.

The dose of fusion protein effective to normalize a patient's blood glucose will depend on a number of factors, among which are included, without limitation, the subject's sex, weight and age, the severity of inability to regulate blood glucose, the route of administration and bioavailability, the pharmacokinetic profile of the fusion protein, the potency, and the formulation. Doses may be in the range of 0.01 to 1 mg/kg body weight, preferably in the range of 0.05 to 0.5 mg/kg body weight.

It is preferable that the fusion proteins be administered either once every two weeks or once a week. Depending on the disease being treated, it may be necessary to administer the fusion protein more frequently such as two to three time per week.

The present invention will now be described only by way of non-limiting example with reference to the following Examples.

### EXAMPLES

### In vitro GLP-1 receptor activation assay:

HEK-293 cells expressing the human GLP-1 receptor, using a CRE-BLAM system, are seeded at 20,000 to 40,000 cells/well/100 µl DMEM medium with 10%FBS into a poly-d-lysine coated 96 well black, clear-bottom plate. The day after seeding, the medium is flicked off and 80 µl plasma-free DMEM medium is added. On the third day after seeding, 20 µl of plasma-free DMEM medium with 0.5% BSA containing different concentrations of various GLP-1-Fc fusion protein is added to each well to generate a dose response curve. Generally, fourteen dilutions containing from 3 nanomolar to 30 nanomolar or GLP-1 Fc fusion protein are used to generate a dose response curve from which EC₅₀ values can be determined. After 5 hours of incubation with the fusion protein, 20 µl of β-lactamase substrate (CCF2/AM, PanVera LLC) is added and incubation continued for 1 hour at which time fluorescence is determined on a cytofluor. The assay is further described in Zlokarnik, et al. (1998), Science, 278:84-88.

### In vitro GLP-1 receptor activation assay:

HEK-293 cells stably expressing the human GLP-1 receptor, using a CRE-Luciferase system, are seeded at 30,000 cells/well/80 µl low serum DMEM F12 medium into 96 well plates. The day after seeding, 20 µl aliquots of test protein dissolved in 0.5% BSA are mixed and incubated with the cells for 5 hours. Generally 12 dilutions containing from 3 pM to 3 nM are prepared at a 5X concentration for each test protein before addition to the cells to generate a dose response curve from which EC₅₀ values are determined. After incubation, 100 µl of Luciferase reagent is added directly to each plate and mixed gently for 2 minutes. Plates are placed in a Tri-lux luminometer and light output resulting from luciferase expression is calculated.

### Effect of Tween, NaCl, and pH on stability of Gly⁸-Glu²²-GLP-1(7-37)-1L-IgG4 (S228P, F234A, L235A):

The effects of tween-20 (± 0.01 %), NaCl (0,150, and 500mM), and pH (6.5, 7.5, and 8.5) on the chemical stability of 0.5mg/mL Gly⁸-Glu²²-GLP-1(7-37)-1L-IgG4 (S228P, F234A, L235A) are determined. A full matrix of these conditions are prepared by mixing a respective 2x buffer stock with a 1mg/mL Gly⁸-Glu²²-GLP-1(7-37)-1L-IgG4 (S228P, F234A, L235A) solution dissolved in 20mM phosphate, 20mM Tris, pH 7.5. The final solution conditions are terminally filtered through 0.2micron Millex-GV filters into HPLC vials and incubated at 37°C. Periodically time points are removed and analyzed by size-exclusion chromatography, reversed-phase chromatography, and gel electrophores.

Identification of formulation components for samples A-R (all contained 0.5mg/mL Gly⁸-Glu²²-GLP-1(7-37)-1L-IgG4 (S228P, F234A, L235A), 20mM phosphate, and 20mM Tris)

| Formulation code | Tween-20 (%) | NaCl (mM) | pH |
|---|---|---|---|
| A | 0 | 0 | 6.5 |
| B | 0 | 0 | 7.5 |
| C | 0 | 0 | 8.5 |
| D | 0 | 150 | 6.5 |
| E | 0 | 150 | 7.5 |
| F | 0 | 150 | 8.5 |
| G | 0 | 500 | 6.5 |
| H | 0 | 500 | 7.5 |
| I | 0 | 500 | 8.5 |
| J | 0.01 | 0 | 6.5 |
| K | 0.01 | 0 | 7.5 |
| L | 0.01 | 0 | 8.5 |
| M | 0.01 | 150 | 6.5 |
| N | 0.01 | 150 | 7.5 |
| O | 0.01 | 150 | 8.5 |
| P | 0.01 | 500 | 6.5 |
| Q | 0.01 | 500 | 7.5 |
| R | 0.01 | 500 | 8.5 |

Change in percent main peak at 37°C for various GLP-Fc solution conditions as monitored by size-exclusion HPLC

| Formulation code--> | **A** | **B** | **C** | **D** | **E** | **F** |
|---|---|---|---|---|---|---|
| Time (weeks @ 37oC) | | | | | | |
| 0 | 98.96 | 98.96 | 98.96 | 98.96 | 98.96 | 98.96 |
| 0.57 | 98.87 | 98.73 | 98.95 | 98.89 | 98.89 | 98.58 |
| 1 | 98.36 | 98.63 | 98.69 | 98.39 | 98.4 | 98.48 |
| 2 | 97.9 | 98.39 | 98.53 | 98.09 | 98.2 | 98.25 |
| 3 | 97.77 | 98.39 | 98.74 | 98.01 | 97.94 | 98.32 |

| Formulation code--> | **G** | **H** | **I** | **J** | **K** | **L** |
|---|---|---|---|---|---|---|
| Time (weeks @ 37oC) | | | | | | |
| 0 | 98.96 | 98.96 | 98.96 | 98.96 | 98.96 | 98.96 |
| 0.57 | 98.85 | 98.69 | 98.37 | 98.67 | 98.42 | 98.63 |
| 1 | 98.53 | | 98.5 | 98.13 | 98.2 | 98.33 |
| 2 | 98.27 | 98.02 | 98.28 | 97.54 | 97.81 | 98.13 |
| 3 | 98.19 | 97.96 | 98.22 | 97.42 | 97.74 | 98.15 |

| Formulation code--> | **M** | **N** | **O** | **P** | **Q** | **R** |
|---|---|---|---|---|---|---|
| Time (weeks @ 37oC) | | | | | | |
| 0 | 98.96 | 98.96 | 98.96 | 98.96 | 98.96 | 98.96 |
| 0.57 | 98.25 | 98.24 | 97.91 | 98.15 | 98.15 | 97.79 |
| 1 | 97.78 | 97.85 | 98 | 97.88 | 97.8 | 97.6 |
| 2 | 97.28 | 97.48 | 97.66 | 97.73 | 97.81 | 97.47 |
| 3 | 96.94 | 97.18 | 97.52 | 97.49 | 97.53 | 97.22 |

Change in percent main peak at 37°C for various GLP-Fc solution conditions as monitored by reversed-phase HPLC

| Formulation code--> | **A** | **B** | **C** | **D** | **E** | **F** |
|---|---|---|---|---|---|---|
| Time (weeks @ 37oC) | | | | | | |
| 0 | 78.2 | 78.2 | 78.2 | 78.2 | 78.2 | 78.2 |
| 0.57 | 77.3 | 77.1 | 75.8 | 77.9 | 75.3 | 76.4 |
| 1 | 70.2 | 69.4 | 69.2 | 73.4 | 73.6 | 69.1 |
| 2 | 67.2 | 66.1 | 62 | 73.3 | 67.5 | 63.6 |
| 3 | 65.2 | 62.6 | 57.3 | 66 | 61.2 | 58.8 |

| Formulation code--> | **G** | **H** | **I** | **J** | **K** | **L** |
|---|---|---|---|---|---|---|
| Time (weeks @ 37oC) | | | | | | |
| 0 | 78.2 | 78.2 | 78.2 | 78.2 | 78.2 | 78.2 |
| 0.57 | 77.4 | 76.9 | 75.8 | 74.9 | 75.3 | 75.4 |
| 1 | 72.6 | 73.3 | 72.5 | 69.5 | 69 | 70.7 |
| 2 | 73.2 | 66.6 | 65.8 | 65.4 | 63.9 | 61.96 |
| 3 | 64.8 | 62.4 | 58.9 | 63.6 | 60.4 | 57.7 |

| Formulation code--> | **M** | **N** | **O** | **P** | **Q** | **R** |
|---|---|---|---|---|---|---|
| Time (weeks @ 37oC) | | | | | | |
| 0 | 78.2 | 78.2 | 78.2 | 78.2 | 78.2 | 78.2 |
| 0.57 | 76.2 | 75.9 | 73.9 | 75 | 72.9 | 75.3 |
| 1 | 71.4 | 72.3 | 69.8 | 75.7 | 71.2 | 71 |
| 2 | 67.5 | 63.5 | 59.4 | 65.2 | 68.5 | 61.4 |

Linear rate of degradation (percent change in main peak per week upon 37°C incubation) as monitored by size-exclusion or reversed-phase HPLC.

| | Percent change in main peak per week upon 37oC incubation | |
|---|---|---|
| Formulation code | size-exclusion data | reversed-phase data |
| A | -0.44 | -9.8 |
| B | -0.19 | -10.8 |
| C | -0.1 | -11.2 |
| D | -0.35 | -5.8 |
| E | -0.36 | -5.9 |
| F | -0.2 | -11.2 |
| G | -0.28 | -6.9 |
| H | -0.36 | -6.1 |
| I | -0.2 | -7.2 |
| J | -0.55 | -10.9 |
| K | -0.39 | -11.5 |
| L | -0.27 | -9.4 |
| M | -0.65 | -8.5 |
| N | -0.56 | -7.4 |
| O | -0.4 | -10.6 |
| P | -0.42 | -3.4 |
| Q | -0.4 | -9.2 |
| R | -0.47 | -9.1 |

### Size-Exclusion HPLC method:

Samples are injected on a Tosohas TSK3000-SW-XL (5 micron, 7.8x300mm) column pre-equilibrated in 1x PBS, pH 7.4 plus 10% acetonitrile (v/v) mobile buffer (A-pump buffer). A-buffer is flowing at 0.5mL/min isocratic and protein was detected at 214nm.

### Reversed-Phase HPLC method:

Samples are injected on a Zorbax 300SB-C8 (4.6x50mm) column pre-equilibrated in 0.1% TFA (v/v) mobile buffer (A-pump buffer). B-pump buffer consists of 0.085% TFA in acetonitrile (v/v). The following gradient is run and material was detected at 214nm.

| Time | % B-pump buffer |
|---|---|
| 0 | 30 |
| 5 | 30 |
| 35 | 45 |
| 40 | 90 |
| 45 | 90 |
| 50 | 25 |
| 65 | 25 |

### Gel Electrophoresis method:

Time points are diluted with 4x NuPage LDS sample buffer (1x final conc) and heated at 95°C for 5 minutes. The cooled samples were loaded on a 4-12% bis-tris NuPage gel. The gel is run at constant voltage (200 volts) for 50-60min in MOPS buffer and stained with SimplyBlue SafeStain.

### Stability of Gly⁸-Glu²²-GLP-1(7-37)-1L-IgG4 (S228P, F234A, L235A) and effect of Tween-20:

Stability of Gly⁸-Glu2²-GLP-1(7-37)-1L-IgG4 (S228P, F234A, L235A) at 0.3mg/mL in 1x PBS at pH 7.5 is evaluated by monitoring the increase in turbidity at 350nm under accelerated stress conditions (stirring with hydrophobic stir bar in PBS at 37oC).

The effect of Tween-20 on reducing the aggregation

| Solution Additive | Time for turbidity to increase ≥ 0.05 OD at 350nm |
|---|---|
| None | < 6-hr |
| 0.001 % Tween-20 | 16-hr |
| 0.005% Tween-20 | 27-hr |
| 0.01 % Tween-20 | > 65-hr |
| 0.02% Tween-20 | > 65-hr |
| 0.05% Tween-20 | > 65-hr |

### Solubility of Gly⁸-Glu²²-GLP-1(7-37)-1L-IgG4 (S228P, F234A, L235A) as a function of solution pH:

Solubility of Gly⁸-Glu²²-GLP-1(7-37)-1L-IgG4 (S228P, F234A, L235A) as a function of pH is determined by diluting a stock solution with various buffered solution at differ pH values, allowing the solution to equilibrate, centrifugating away any precipitated material, then analyzing the supernant for remaining soluble protein concentration. Stock protein frozen in PBS, is dialyzed against water at approximately pH 8. After dialysis the protein stock was filtered through a 0.22micron Millex-GV filter and concentrated to ∼12mg/mL using a rinsed Ultrafree centrifugal device (4ml, 5000 dalton MWCO, at 7000xG). This concentrated solution is diluted 1:1 with 2x stock buffer solutions (final volume of 50uL in 0.5mL eppendorf tubes) and is allowed to equilibrate for 15-30 minutes at room temperature. The solution is centrifuged (14,000xG for 5 minutes) to pellet any insoluble material. 10uL of the supernant is removed and combined with 40uL of the pH 8 water. Two replicates are performed for each sample. The concentration of soluble GLP-Fc is determined by reversed-phase HPLC against a standard curve.

Effect of solution conditions on solubility

| | Protein Concentration (mg/mL) | |
|---|---|---|
| Formulation condition | Average | Stdev (n=2) |
| 20mM Citrate, pH 3 | 5.8 | 0.4 |
| 20mM Citrate, pH 4 | 4.1 | 0.3 |
| 20mM Citrate, pH 5 | 4.5 | 0.0 |
| 20mM Citrate, pH 6 | 5.5 | 0.0 |
| 20mM Phosphate, pH 6 | 5.8 | 0.1 |
| 20mM Phosphate, pH 7 | 5.8 | 0.4 |
| 20mM Phosphate, pH 7 (replicate #1) | 6.0 | 0.1 |
| 20mM Phosphate, pH 7 (replicate #2) | 5.8 | 0.0 |
| 20mM Phosphate, pH 8 | 5.5 | 0.0 |
| 20mM Tris, pH 8 | 6.1 | 0.1 |
| 20mM Citrate, 150mM NaCl, pH 3 | 0.4 | 0.0 |
| 20mM Citrate, 150mM NaCl, pH 4 | 2.8 | 0.1 |
| 20mM Citrate, 150mM NaCl, pH 5 | 6.0 | 0.0 |
| 20mM Citrate, 150mM NaCl, pH 6 | 5.3 | 0.1 |
| 20mM Phosphate, 150mM NaCl, pH 7 | 5.9 | 0.0 |
| 20mM Tris, 150mM NaCl, pH 8 | 6.0 | 0.1 |
| 20mM Citrate, 3mg/mL m-Cresol, pH 3 | 5.8 | 0.0 |
| 20mM Citrate, 3mg/mL m-Cresol, pH 4 | 1.0 | 0.0 |
| 20mM Citrate, 3mg/mL m-Cresol, pH 5 | 1.3 | 0.0 |
| 20mM Citrate, 3mg/mL m-Cresol, pH 6 | 5.5 | 0.1 |
| 20mM Phosphate, 3mg/mL m-Cresol, pH 7 | 6.2 | 0.1 |
| 20mM Tris, 3mg/mL m-Cresol, pH 8 | 6.0 | 0.0 |

### Effect of solution pH on stability as monitored by reversed-phase HPLC:

Gly⁸-Glu²²-GLP-1(7-37)-1L-IgG4 (S228P, F234A, L235A) is dialyzed against water adjusted to approximately pH 8. After dialysis this protein stock is filtered (0.2 micron Millex-GV) and concentrated to >2mg/mL using a rinsed Ultrafree centrifugal device. The final protein concentration is determined by UV and various formulations are made by diluted 2x formulation buffer stocks to 1x keeping the final protein concentration at 1mg/mL. The solution pH is checked and adjusted if needed. These solutions are terminally filtered (0.22micon Millex-GV, 4mm) in sterilized 1.8mL HPLC vials with screw caps. The vials are placed at 37°C and time points are pulled and analyzed by reversed-phase, size-exclusion, and gel electrophoresis. The change in percent main-peak as monitored by size-exclusion chromatography and reversed-phase HPLC are presented. The linear degradation rates obtained by fitting a plot of incubation time at 37oC with main peak purity for the size-exclusion and reversed-phase HPLC data is tabulated.

Identification of formulation parameters.

| Formulation code | PH | Buffer (10mM) | Additive |
|---|---|---|---|
| 3C | 3.0 | Citrate | none |
| 4C | 4.0 | Citrate | none |
| 5C | 5.0 | Citrate | none |
| 6C | 6.0 | Citrate | none |
| 6CN | 6.0 | Citrate | 150mM NaCl |
| 6CC | 6.0 | Citrate | 3mg/mL m-Cresol |
| 6P | 6.0 | Phosphate | none |
| 7P-R1 | 7.0 | Phosphate | None, replicate #1 |
| 7P-R2 | 7.0 | Phosphate | None, replicate #2 |
| 7P-R3 | 7.0 | Phosphate | None, replicate #3 |
| 7PN | 7.0 | Phosphate | 150mM NaCl |
| 7PC | 7.0 | Phosphate | 3mg/mL m-Cresol |
| 8P | 8.0 | Phosphate | none |
| 8T | 8.0 | Tris | none |

The change in percent main-peak upon storage at 37°C. Data is analyzed by size-exclusion chromatography.

| Time (weeks) | 3C | 4C | 5C | 6C | 6CN | 6CC | 6P |
|---|---|---|---|---|---|---|---|
| 0.43 | 43.81 | 0 | 94.27 | 95.74 | 94.37 | 94.45 | 94.7 |
| 1 | 40.13 | 0 | 93.14 | 94.73 | 92.98 | 92.79 | 93.85 |
| 2.43 | 36.26 | 0 | 91.72 | 94.22 | 92.29 | 90.77 | 93.1 |
| 3.43 | 35.85 | 0 | 91.27 | 93.84 | 91.51 | 89.44 | 92.51 |

| Time (weeks) | 7P-R1 | 7P-R2 | 7P-R3 | 7PN | 7PC | 8P | 8T |
|---|---|---|---|---|---|---|---|
| 0.43 | 95.98 | 95.95 | 96.02 | 93.93 | 95.86 | 95.89 | 97.08 |
| 1 | 95.22 | 95.03 | 95.22 | 92.95 | 95.18 | 95.26 | 96.92 |
| 2.43 | 94.71 | 94.5 | 94.84 | 91.76 | 94.76 | 94.94 | 97.05 |
| 3.43 | 94.05 | 93.65 | 94.09 | 91.24 | 94.02 | 94.67 | 97.02 |

The change in percent main-peak upon storage at 37°C. Data is analyzed by reversed-phase chromatography.

| Time (weeks) | 3C | 4C | 5C | 6C | 6CN | 6CC | 6P |
|---|---|---|---|---|---|---|---|
| 0.43 | 34.04 | 39.35 | 51.93 | 57.96 | 57.29 | 58.42 | 55.76 |
| 1 | 26.64 | 39.48 | 49.85 | 58.49 | 53.32 | 55.87 | |
| 2.43 | 33.09 | 40.36 | 44.61 | 52.05 | 51.24 | 51.34 | 49.44 |
| 3.43 | 28.17 | 28.51 | 36.78 | 48.4 | 50.43 | 50.42 | 49.84 |

| Time (weeks) | 7P-R1 | 7P-R2 | 7P-R3 | 7PN | 7PC | 8P | 8T |
|---|---|---|---|---|---|---|---|
| 0.43 | 55.74 | 55.36 | 55.01 | 56.09 | 58.67 | 49.14 | 54.44 |
| 1 | 53.72 | 54 | 53.22 | 52.4 | 55.96 | 46.84 | 54.48 |
| 2.43 | 45.7 | 47 | 45.97 | 47.07 | 51.49 | 38.7 | 51.22 |
| 3.43 | 44.16 | 42 | 43.22 | 42.62 | 49.74 | 34.25 | 47.89 |

Rate of degradation at 37oC as monitored by size-exclusion or reversed-phase HPLC.

| | Percent change in main peak per week upon 37oC incubation | |
|---|---|---|
| Formulation code | size-exclusion data | reversed-phase data |
| 3C | -2.6 | -0.71 |
| 4C | precipitated | -3.0 |
| 5C | -0.98 | -4.9 |
| 6C | -0.57 | -3.5 |
| 6P | -0.68 | -2.1 |
| 7P-R1 | -0.58 | -4.1 |
| 7P-R2 | -0.69 | -4.6 |
| 7P-R3 | -0.57 | -4.1 |
| 8P | -0.36 | -5.1 |
| 8T | 0.003 | -2.2 |
| 6CN | -0.85 | -2.1 |
| 6CC | -1.6 | -2.7 |
| 7PN | -0.87 | -4.3 |
| 7PC | -0.55 | -3.0 |

## Claims

1. A stable solution formulation comprising a therapeutically effective amount of a GLP-1-Fc fusion at a pH of between about pH 6 and about pH 8.5
wherein the GLP-1-Fc fusion comprises a GLP-1 analog of a sequence selected from: 
a) (SEQ ID NO:1)
His-Xaa8-Glu-Gly-Thr-Phe-Thr-Ser-Asp-Val-Ser-Ser-Tyr-Leu-Glu-Glu- Gln-Ala-Ala-
Lys-Glu-Phe-Ile-Ala-Trp-Leu-Val-Lys-Gly-Gly-Gly
wherein Xaa8 is selected from Gly and Val;
 
b) (SEQ ID NO:2)
His-Xaa8-Glu-Gly-Thr-Phe-Thr-Ser-Asp-Val-Ser-Ser-Tyr-Leu-Glu-Glu- Gln-Ala-Ala-
Lys-Glu-Phe-Ile-Ala-Trp-Leu-Lys-Asn-Gly-Gly-Gly
wherein Xaa8 is selected from Gly and Val;
 
c) (SEQ ID NO:3)
His-Xaa8-Glu-Gly-Thr-Phe-Thr-Ser-Asp-Val-Ser-Ser-Tyr-Leu-Glu-Glu- Gln-Ala-Ala-
Lys-Glu-Phe-Ile-Ala-Trp-Leu-Val-Lys-Gly-Gly-Pro
wherein Xaa8 is selected from Gly and Val;
 
d) (SEQ ID NO:4)
His-Xaa8-Glu-Gly-Thr-Phe-Thr-Ser-Asp-Val-Ser-Ser-Tyr-Leu-Glu-Glu- Gln- Ala-Ala-
Lys-Glu-Phe-Ile-Ala-Trp-Leu-Lys-Asn-Gly-Gly-Pro
wherein Xaa8  is selected from Gly and Val;
 
e) (SEQ ID NO:5)
His-Xaa8-Glu-Gly-Thr-Phe-Thr-Ser-Asp-Val-Ser-Ser-Tyr-Leu-Glu-Glu- Gln-Ala-Ala-
Lys-Glu-Phe-Ile-Ala-Trp-Leu-Val-Lys-Gly-Gly
wherein Xaa8 is selected from Gly and Val;
 
f) (SEQ ID NO:6)
His-Xaa8-Glu-Gly-Thr-Phe-Thr-Ser-Asp-Val-Ser-Ser-Tyr-Leu-Glu-Glu- Gln-Ala-Ala-
Lys-Glu-Phe-Ile-Ala-Trp-Leu-Lys-Asn-Gly-Gly
 wherein Xaa₈ is selected from Gly and Val;
an Fc portion of an immunoglobulin of SEQ ID NO:7 
Ala-Glu-Ser-Lys-Tyr-Gly-Pro-Pro-Cys-Pro-Pro-Cys-Pro-Ala-Pro-
Xaa16-Xaa17-Xaa18-Gly-Gly-Pro-Ser-Val-Phe-Leu-Phe-Pro-Pro-Lys-Pro-
Lys-Asp-Thr-Leu-Met-Ile-Ser-Arg-Thr-Pro-Glu-Val-Thr-Cys-Val-
Val-Val-Asp-Val-Ser-Gln-Glu-Asp-Pro-Glu-Val-Gln-Phe-Asn-Trp-
Tyr-Val-Asp-Gly-Val-Glu-Val-His-Asn-Ala-Lys-Thr-Lys-Pro-Arg-
Glu-Glu-Gln-Phe-Xaa80-Ser-Thr-Tyr-Arg-Val-Val-Ser-Val-Leu-Thr-
Val-Leu-His-Gln-Asp-Trp-Leu-Asn-Gly-Lys-Glu-Tyr-Lys-Cys-Lys-
Val-Ser-Asn-Lys-Gly-Leu-Pro-Ser-Ser-Ile-Glu-Lys-Thr-Ile-Ser-
Lys-Ala-Lys-Gly-Gln-Pro-Arg-Glu-Pro-Gln-Val-Tyr-Thr-Leu-Pro-
Pro-Ser-Gln-Glu-Glu-Met-Thr-Lys-Asn-Gln-Val-Ser-Leu-Thr-Cys-
Leu-Val-Lys-Gly-Phe-Tyr-Pro-Ser-Asp-Ile-Ala-Val-Glu-Trp-Glu-
Ser-Asn-Gly-Gln-Pro-Glu-Asn-Asn-Tyr-Lys-Thr-Thr-Pro-Pro-Val-
Leu-Asp-Ser-Asp-Gly-Ser-Phe-Phe-Leu-Tyr-Ser-Arg-Leu-Thr-Val-
Asp-Lys-Ser-Arg-Trp-Gln-Glu-Gly-Asn-Val-Phe-Ser-Cys-Ser-Val-
Met-His-Glu-Ala-Leu-His-Asn-His-Tyr-Thr-Gln-Lys-Ser-Leu-Ser-
Leu-Ser-Leu-Gly-Xaa230
 wherein:
Xaa at position 16 is Pro or Glu;
Xaa at position 17 is Phe, Val, or Ala;
Xaa at position 18 is Leu, Glu, or Ala;
Xaa at position 80 is Asn or Ala; and
Xaa at position 230 is Lys or is absent
and a peptide linker of SEQ ID NO:8
Gly-Gly-Gly-Gly-Ser-Gly-Gly-Gly-Gly-Ser-Gly-Gly-Gly-Gly-Ser
wherein the N-terminal glycine of the peptide linker is directly fused to the C-terminal amino acid residue of the GLP- I analog and the C-terminal serine of the peptide linker is directly fused to N-terminal alanine of the Fc portion.

2. A stable solution formulation according to claim 1, wherein the pH is between about pH 6 and about pH 7.5.

3. A stable solution formulation according to claim 2, wherein the pH is between about pH 6 and about pH 7.

4. A stable solution formulation according to claim 3, wherein the pH is between about pH 6 and about pH 6.5.

5. A stable solution formulation according to claim 4, wherein the pH is about pH 6.

6. A stable solution formulation according to claim 4, wherein the pH is about pH 6.5.

7. A stable solution formulation according to any one of the preceding claims, wherein the formulation further comprises polyoxyethylene (20) sorbitan monolaurate

8. A stable solution formulation according to any one of claims 1 to 6, wherein the formulation further comprises polyoxyethylene (20) sorbitan monooleate

9. Astable solution formulation according to any one of claims 1 to 8, wherein the formulation further comprises NaCl.

10. A stable solution formulation according to any one of claims 1 to 9, wherein the formulation further comprises m-Cresol.

11. A stable solution formulation according to any one of claims 1 to 6, 7, 9 and 10, wherein the formulation further comprises polyoxyethylene (20) sorbitan monolaurate, NaCl, and m-Cresol.

12. A stable solution formulation according to any one of claims 1 to 6, 8, 9 and 10, wherein the formulation further comprises polyoxyethylene (20) sorbitan monooleate, NaCl, and m-Cresol.

13. A stable solution formulation according to any one of the preceding claims for use as a medicament.

14. A stable solution formulation according to any one of claims 1 to 12 for use in the treatment of insulin dependent and non-insulin dependent diabetes mellitus and obesity.


SEQUENCE LISTING

<110>  Eli Lilly and Company

<120>  GLP-1 Analog Fusion Protein Formulations

<130>  X-17003

<150>  60/641690
<151>  2004-12-22

<160>  17

<170>  PatentIn version 3.3

<210>  1
<211>  31
<212>  PRT
<213>  Artificial

<220>
<223>  Synthetic Construct


<220>
<221>  MISC_FEATURE
<222>  (2)..(2)
<223>  Xaa at position 2 is selected from Gly and Val

<400>  1

His Xaa Glu Gly Thr Phe Thr Ser Asp Val Ser Ser Tyr Leu Glu Glu
1               5                   10                  15


Gln Ala Ala Lys Glu Phe Ile Ala Trp Leu Val Lys Gly Gly Gly
            20                  25                  30


<210>  2
<211>  31
<212>  PRT
<213>  Artificial

<220>
<223>  Synthetic Construct


<220>
<221>  MISC_FEATURE
<222>  (2)..(2)
<223>  Xaa at position 2 is selected from Gly and Val

<400>  2

His Xaa Glu Gly Thr Phe Thr Ser Asp Val Ser Ser Tyr Leu Glu Glu
1               5                   10                  15


Gln Ala Ala Lys Glu Phe Ile Ala Trp Leu Lys Asn Gly Gly Gly
            20                  25                  30


<210>  3

<211>  31
<212>  PRT
<213>  Artificial

<220>
<223>  Synthetic Construct


<220>
<221>  MISC_FEATURE
<222>  (2)..(2)
<223>  Xaa at position 2 is selected from Gly and Val

<400>  3

His Xaa Glu Gly Thr Phe Thr Ser Asp Val Ser Ser Tyr Leu Glu Glu
1               5                   10                  15


Gln Ala Ala Lys Glu Phe Ile Ala Trp Leu Val Lys Gly Gly Pro
            20                  25                  30


<210>  4
<211>  31
<212>  PRT
<213>  Artificial

<220>
<223>  Synthetic Construct


<220>
<221>  MISC_FEATURE
<222>  (2)..(2)
<223>  Xaa at position 2 is selected from Gly and Val

<400>  4

His Xaa Glu Gly Thr Phe Thr Ser Asp Val Ser Ser Tyr Leu Glu Glu
1               5                   10                  15


Gln Ala Ala Lys Glu Phe Ile Ala Trp Leu Lys Asn Gly Gly Pro
            20                  25                  30


<210>  5
<211>  30
<212>  PRT
<213>  Artificial

<220>
<223>  Synthetic Construct


<220>
<221>  MISC_FEATURE
<222>  (2)..(2)
<223>  Xaa at position 2 is selected from Gly and Val

<400>  5

His Xaa Glu Gly Thr Phe Thr Ser Asp Val Ser Ser Tyr Leu Glu Glu

1                   5                   10                  15

Gln Ala Ala Lys Glu Phe Ile Ala Trp Leu Val Lys Gly Gly
            20                  25                  30

<210>  6
<211>  30
<212>  PRT
<213>  Artificial

<220>
<223>  Synthetic Construct

<220>
<221>  MISC_FEATURE
<222>  (2)..(2)
<223>  Xaa at position 2 is selected from Gly and Val

<400>  6

His Xaa Glu Gly Thr Phe Thr Ser Asp Val Ser Ser Tyr Leu Glu Glu
1               5                   10                  15

Gln Ala Ala Lys Glu Phe Ile Ala Trp Leu Lys Asn Gly Gly
            20                  25                  30

<210>  7
<211>  230
<212>  PRT
<213>  Artificial

<220>
<223>  Synthetic Construct

<220>
<221>  MISC_FEATURE
<222>  (16)..(16)
<223>  Xaa at position 16 is Pro or Glu

<220>
<221>  MISC_FEATURE
<222>  (17)..(17)
<223>  Xaa at position 17 is Phe, Val, or Ala

<220>
<221>  MISC_FEATURE
<222>  (18)..(18)
<223>  Xaa at position 18 is Leu, Glu, or Ala

<220>
<221>  MISC_FEATURE
<222>  (80)..(80)
<223>  Xaa at position 80 is Asn or Ala

<220>
<221>  MISC_FEATURE
<222>  (230)..(230)
<223>  Xaa at position 230 is Lys or is absent

<400>  7

Ala Glu Ser Lys Tyr Gly Pro Pro Cys Pro Pro Cys Pro Ala Pro Xaa
1               5                   10                  15

Xaa Xaa Gly Gly Pro Ser Val Phe Leu Phe Pro Pro Lys Pro Lys Asp
            20                  25                  30

Thr Leu Met Ile Ser Arg Thr Pro Glu Val Thr Cys Val Val Val Asp
        35                  40                  45

Val Ser Gln Glu Asp Pro Glu Val Gln Phe Asn Trp Tyr Val Asp Gly
    50                  55                  60

Val Glu Val His Asn Ala Lys Thr Lys Pro Arg Glu Glu Gln Phe Xaa
65                  70                  75                  80

Ser Thr Tyr Arg Val Val Ser Val Leu Thr Val Leu His Gln Asp Trp
                85                  90                  95

Leu Asn Gly Lys Glu Tyr Lys Cys Lys Val Ser Asn Lys Gly Leu Pro
            100                 105                 110

Ser Ser Ile Glu Lys Thr Ile Ser Lys Ala Lys Gly Gln Pro Arg Glu
        115                 120                 125

Pro Gln Val Tyr Thr Leu Pro Pro Ser Gln Glu Glu Met Thr Lys Asn
    130                 135                 140

Gln Val Ser Leu Thr Cys Leu Val Lys Gly Phe Tyr Pro Ser Asp Ile
145                 150                 155                 160

Ala Val Glu Trp Glu Ser Asn Gly Gln Pro Glu Asn Asn Tyr Lys Thr
                165                 170                 175

Thr Pro Pro Val Leu Asp Ser Asp Gly Ser Phe Phe Leu Tyr Ser Arg
            180                 185                 190

Leu Thr Val Asp Lys Ser Arg Trp Gln Glu Gly Asn Val Phe Ser Cys
        195                 200                 205

Ser Val Met His Glu Ala Leu His Asn His Tyr Thr Gln Lys Ser Leu
    210                 215                 220

Ser Leu Ser Leu Gly Xaa
225                 230

<210>  8

<211>  15
<212>  PRT
<213>  Artificial

<220>
<223>  Synthetic Construct

<400>  8

Gly Gly Gly Gly Ser Gly Gly Gly Gly Ser Gly Gly Gly Gly Ser
1               5                   10                  15


<210>  9
<211>  31
<212>  PRT
<213>  Artificial

<220>
<223>  Synthetic Construct

<400>  9

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly
            20                  25                  30


<210>  10
<211>  7
<212>  PRT
<213>  Artificial

<220>
<223>  Synthetic Construct

<400>  10

Trp Leu Val Lys Gly Gly Gly
1               5


<210>  11
<211>  7
<212>  PRT
<213>  Artificial

<220>
<223>  Synthetic Construct

<400>  11

Trp Leu Lys Asn Gly Gly Gly
1               5


<210>  12
<211>  7
<212>  PRT
<213>  Artificial

<220>
<223>  Synthetic Construct

<400>  12

Trp Leu Val Lys Gly Gly Pro
1               5


<210>  13
<211>  7
<212>  PRT
<213>  Artificial

<220>
<223>  Synthetic Construct

<400>  13

Trp Leu Lys Asn Gly Gly Pro
1               5


<210>  14
<211>  6
<212>  PRT
<213>  Artificial

<220>
<223>  Synthetic Construct

<400>  14

Trp Leu Val Lys Gly Gly
1               5


<210>  15
<211>  6
<212>  PRT
<213>  Artificial

<220>
<223>  Synthetic Construct

<400>  15

Trp Leu Lys Asn Gly Gly
1               5


<210>  16
<211>  6
<212>  PRT
<213>  Artificial

<220>
<223>  Synthetic Construct

<400>  16

Pro Pro Cys Pro Ser Cys
1               5

<210>  17
<211>  22
<212>  PRT
<213>  Artificial

<220>
<223>  Synthetic Construct

<400>  17

Gly Ser Gly Gly Gly Gly Ser Gly Gly Gly Gly Ser Gly Gly Gly Gly
1               5                   10                  15

Ser Gly Gly Gly Gly Ser
            20